# EUROPEAN PATENT APPLICATION

(11) **EP 4 502 085 A1**
(43) Date of publication of application: **05.02.2025**
(21) Application number: 23779904.4
(22) Date of filing: 22.03.2023
(51) Int. Cl.: C09D 5/03, A61Q 1/00, A61Q 1/04, A61Q 1/06, A61Q 1/08, A61Q 1/10, A61Q 3/00, A61Q 17/04, A61Q 19/00, A61Q 19/10, C09D 183/04, C09D 7/61, C09D 7/62, A61K 8/02, A61K 8/29, C09C 1/36, C09C 3/06, C09C 3/08, C09C 3/10

(54) **POWDER OF TITANIUM(IV) OXIDE COMPOSED OF PARTICLES HAVING PEANUT-LIKE TWIN SHAPE, AND COSMETIC CONTAINING SAME**

(30) Priority: 31.03.2022 JP 2022060113
(71) Applicant: Titan Kogyo Kabushiki Kaisha, Ube-shi Yamaguchi 755-8567 (JP)
(72) Inventor: NADA, Chihiro, Ube-shi, Yamaguchi 755-8567 (JP); SHIMOMURA, Naotaka, Ube-shi, Yamaguchi 755-8567 (JP); MINO, Wataru, Ube-shi, Yamaguchi 755-8567 (JP)
(74) Representative: Cabinet Beau de Loménie
(86) International application number: PCT/JP2023/011099
(87) International publication number: WO 2023/189890

(57) **Abstract**

Provided is a titanium oxide with which it is possible to provide a cosmetic capable of realizing a uniform cosmetic film and creating a natural bare skin feel by having light spreading, soft focus properties, and moderate concealing power. Titanium (IV) oxide configured from particles characterized by having a peanut-like twin shape in which the major axis diameter, which is the longest part of a single particle, is 300-700 nm, the minor axis diameter, which is the largest portion in a direction perpendicular to the major axis, is 100-400 nm, the constriction diameter, which is the length in the direction perpendicular to the major axis at the center of the major axis, is 80-200 nm, and the ratio [constriction diameter]/[minor axis diameter] is 0.9 or less, and a cosmetic containing the titanium (IV) oxide.

## Description

### TECHNICAL FIELD

The present invention relates to a titanium (IV) oxide powder composed of particles characterized by a peanut-like twin shape and a method for producing the same.

The present invention also relates to a cosmetic containing the titanium (IV) oxide powder. Cosmetics containing the titanium (IV) oxide have easy spreadability, soft focus properties, and appropriate hiding power, so that a cosmetic film with excellent uniformity can be produced to exhibit a natural bare skin feel.

### BACKGROUND ART

Conventionally, makeup cosmetics such as foundations that contain a pigment having a large coloring power such as titanium (IV) oxide and a coloring agent such as other inorganic pigments and organic pigments have been used as cosmetics that change the color tone of the skin to cover rubicund face, dullness, age spots, freckles, etc. in the skin, and to exhibit a uniform and beautiful skin in appearance.

Regarding the titanium oxide described above, pigments with high coloring power and hiding power as represented by titanium (IV) oxide are generally used to cover rubicund face, dullness, etc., and to erase unevenness of the skin. In JP S61-118311 A (Patent Literature 1), spherical titanium oxide having a particle size of 0.1 µm to 50 µm is used.

However, in the case of applying makeup to the skin using the foundation containing the titanium oxide, a creaky feel may be caused or a smooth feel of the skin may be lost. Furthermore, under sunlight, a problem that the finish of makeup looks pale and unnatural, which is generally referred to as white cast, due to high reflectance of bluish colors may occur. In order to solve the problem, attempts have been made to set the primary particle diameter to a smaller value.

For example, in JP H09-221411 A (Patent Literature 2) a cosmetic containing titanium dioxide having an average particle size of more than 0.10 µm and 0.14 µm or less is disclosed. Further, in JP H11-158035 (Patent Literature 3), a cosmetic containing titanium oxide having an anatase crystal form with a primary particle size of 0.001 µm or more and 0.15 µm or less and a secondary particle size of 0.6 µm or more and 2.0 µm or less is proposed.

Further, in JP 2000-191325 A (Patent Literature 4), use of aggregates of spherical titanium dioxide as cosmetic is proposed, wherein the aggregates have an apparent average particle diameter of 0.1 µm or more and 3 µm or less, and the aggregates are formed of small spherical particles of titanium dioxide having an average primary particle diameter of 0.01 µm or more and 0.07 µm or less as measured by X-ray diffraction method. Further, it is disclosed that the cosmetic is expected to impart good slipperiness and excellent light stability that conventional titanium dioxide does not have.

In JP 2008-56535 A (Patent Literature 5), the present applicant has proposed rutile-type titanium oxide agglomerated particles with a particle diameter of 0.1 µm or more and 5.0 µm or less formed by aggregating rod-shaped primary particles of titanium oxide to form a fan-shaped titanium oxide, and further agglomerating the fan-shaped titanium oxide, and a cosmetic made of the agglomerated particles. Further, the present applicant has reported that the cosmetic can be applied smoothly to the skin, gives no creaky feel and no rough feel to the skin, and exhibits a natural bare skin feel without white cast, due to moderate coloring power and hiding power.

### CITATION LIST

### PATENT LITERATURE

PTL 1: JP S61-118311 A
PTL 2: JP H09-221411 A
PTL 3: JP H11-158035 A
PTL 4: JP 2000-191325 A
PTL 5: JP 2008-56535 A

### SUMMARY OF INVENTION

### TECHNICAL PROBLEM

However, the cosmetic described in Patent Literature 2 has insufficient spreadability and slippery when applying makeup.

Further, the cosmetic described in Patent Literature 3 has high activity and a strong bluish color due to anatase-type crystal form, as well as insufficient dispersion when compounded into cosmetics.

Further, the cosmetic described in Patent Literature 4 requires use of hydrogen peroxide and an autoclave in production, and production safety and production efficiency were not necessarily satisfied.

The cosmetic described in Patent Literature 5 leaves a slightly pale impression, and there is still room for improvement in hiding power. The present inventors believed that it is important to have an appropriate hiding power in order to stably obtain cosmetics that are free from bluishness without white cast.

In addition to relying on hiding power, the present inventors have focused on means of blurring to make age spots or the like on the skin less noticeable. In other words, the present inventors believed that a titanium oxide pigment with soft focus properties is able to give a natural impression with less occurrence of white cast, without need to increase the hiding power excessively.

Accordingly, an object of the present invention is to provide titanium (IV) oxide particles from which a uniform cosmetic film is formed when used in cosmetics, being capable of exhibiting a natural bare skin feel due to soft focus properties and appropriate hiding power in addition to easy spreadability, and to provide a cosmetic containing the particles.

### SOLUTION TO PROBLEM

As a result of intensive study on the problem, the present inventors have found that a cosmetic that spreads easily, having soft focus properties and appropriate hiding power, capable of exhibiting a natural-looking bare skin feel can be provided with use of a titanium (IV) oxide powder composed of peanut-like twin shaped particles having a major axis diameter, which is the longest part of a single particle, of 300 nm or more and 700 nm or less, a minor axis diameter, which is the maximum part in the direction perpendicular to the major axis, of 100 nm or more and 400 nm or less, a constriction diameter, which is the length in the direction perpendicular to the major axis at the central position of the major axis, of 80 nm or more and 200 nm or less, and a ratio of constriction diameter/minor axis diameter of 0.9 or less. The present invention includes, but is not limited to, the following:
[1] A titanium (IV) oxide powder composed of peanut-like twin shaped particles having a major axis diameter, which is the longest part of a single particle, of 300 nm or more and 700 nm or less, a minor axis diameter, which is the maximum part in the direction perpendicular to the major axis, of 100 nm or more and 400 nm or less, a constriction diameter, which is the length in the direction perpendicular to the major axis at the central position of the major axis, of 80 nm or more and 200 nm or less, and a ratio of constriction diameter/minor axis diameter of 0.9 or less.
[2] The titanium (IV) oxide powder according to item [1], wherein the titanium (IV) oxide is rutile-type titanium oxide.
[3] The titanium (IV) oxide powder according to item [1] or [2], wherein the single particle is formed by aggregation of fine rod-like particles having a long axis diameter of 30 nm or more and 350 nm or less and a short axis diameter of 3 nm or more and 12 nm or less into a peanut-like twin shape.
[4] The titanium (IV) oxide powder according to any one of items [1] to [3], wherein in the case where the powder is stirred at 2000 rpm in a mixed solution of trimethylsiloxysilicic acid and cyclopentane siloxane at a mass ratio of 1:1 as medium to obtain a coating material with a powder concentration of 100 g/kg, and a coating film formed therefrom with a bar coater having a film thickness of 18.4 µm is exposed to C-light at a color temperature of 6774 K by a double beam method, the coating film has a total light transmittance of 45.0% or more and 60.0% or less, and a haze value of 90.0% or more.
[5] The titanium (IV) oxide powder according to any one of items [1] to [4], wherein at least a part of the surface of the particles constituting the powder is coated with a layer of an inorganic substance and/or an organic substance.
[6] The titanium (IV) oxide powder according to item [5], wherein at least a part of the surface of the particles constituting the powder is coated with a layer of an inorganic substance comprising one or more of aluminum, silicon, zinc, titanium, zirconium, iron, cerium, and tin.
[7] The titanium (IV) oxide powder according to item [5], wherein at least a part of the surface of the particles constituting the powder is coated with a layer of an organic substance comprising one or more of a silicone compound, a coupling agent, a fluorine compound, and a fatty acid.
[8] A cosmetic containing the titanium (IV) oxide powder according to any one of items [1] to [7].
[9] A method for producing the titanium (IV) oxide powder according to any one of items [1] to [7] including step 1 of adding hydrochloric acid to a slurry containing an acid-soluble titanium compound, and holding the mixture with a hydrochloric acid concentration controlled to 60 g/L or more and 130 g/L or less at a temperature controlled to 25°C or more and 60°C or less, for 1 hour or more.
[10] The production method according to item [9], wherein in step 1, the hydrochloric acid concentration is controlled to 80 g/L or more and 130 g/L or less, and the temperature is controlled to 35°C or more and 45°C or less.
[11] The production method according to item [9] or [10], further including step 2 of controlling the hydrochloric acid concentration and/or the temperature to higher than those in step 1 within a range of hydrochloric acid concentration of 180 g/L or less and a range of temperature of 100°C or less and holding for 1 hour or more, after step 1.
[12] The production method according to item [11], further including step 3 of firing after step 2.

### ADVANTAGEOUS EFFECTS OF INVENTION

The titanium (IV) oxide particles of the present invention blended in a cosmetic, particularly in a makeup cosmetic, allows a uniform cosmetic film to be formed, so that a natural bare skin feel can be exhibited. Further, a coating film including the titanium (IV) oxide particles of the present invention has soft focus properties and appropriate hiding power.

### BRIEF DESCRIPTION OF DRAWINGS

Fig. 1 is a transmission electron micrograph showing titanium (IV) oxide particles having a peanut-like twin shape obtained in Example 1.
Fig. 2 is a transmission electron micrograph showing titanium (IV) oxide particles having a peanut-like twin shape obtained in Example 2.
Fig. 3 is a transmission electron micrograph showing titanium (IV) oxide particles having a peanut-like twin shape obtained in Example 3.
Fig. 4 is a transmission electron micrograph showing titanium (IV) oxide particles having a peanut-like twin shape obtained in Example 4.
Fig. 5 is a transmission electron micrograph showing titanium (IV) oxide particles having a peanut-like twin shape obtained in Example 5.
Fig. 6 is a transmission electron micrograph showing titanium (IV) oxide particles having a peanut-like twin shape obtained in Example 6.
Fig. 7 is a transmission electron micrograph showing titanium (IV) oxide particles obtained in Comparative Example 1.
Fig. 8 is a transmission electron micrograph showing titanium (IV) oxide particles obtained in Comparative Example 2.

### DESCRIPTION OF EMBODIMENTS

According to the present invention, a powder composed of titanium (IV) oxide particles having a peanut-like twin shape with a major axis diameter and a minor axis diameter in specific ranges and a constriction part at the center, is provided. Since the particles have an appropriate size, the particles blended in a cosmetic allows the cosmetic to spread easily. Further, due to the shape, smoothness, soft focus properties and appropriate hiding power are provided, and when made into a cosmetic film, excellent uniformity can be obtained. Thus, a cosmetic capable of exhibiting a natural-looking bare skin feel can be produced. Also, excellent dispersion is achieved when blended into a cosmetic, and a cosmetic with excellent feel can be provided.

The titanium (IV) oxide particles that constitute the powder of the present invention have a major axis diameter, which is the longest part of a particle, of 300 nm or more and 700 nm or less, a minor axis diameter, which is the maximum part in the direction perpendicular to the major axis, of 100 nm or more and 400 nm or less, a constriction diameter, which is the length in the direction perpendicular to the major axis at the central position of the major axis, of 80 nm or more and 200 nm or less, and a ratio of constriction diameter/minor axis diameter obtained by dividing the constriction diameter by the minor axis diameter, of 0.9 or less. As described above, the particles constituting the powder of the present invention have a peanut-like twin shape with a constriction at the center. In order to obtain a peanut-like twin shape, the diameter of the second largest portion in the direction perpendicular to the long axis direction is preferably larger than the constriction diameter. Within the range, the larger the long axis diameter is, the more transparent cosmetic with natural bare skin feel can be obtained. However, with a major axis diameter of more than 700 nm, the hiding power becomes too small, so that age spots or the like cannot be hidden. Also, with a ratio of constriction diameter/minor axis diameter of more than 0.9, the soft focus properties are lowered. The lower limit of the major axis diameter is more preferably 350 nm or more, still more preferably 400 nm or more, and the upper limit is more preferably 650 nm or less. The lower limit of the minor axis diameter is more preferably 110 nm or more, still more preferably 150 nm or more, and the upper limit is more preferably 360 nm or less. The lower limit of the constriction diameter is more preferably 85 nm or more, still more preferably 100 nm or more, and the upper limit is more preferably 195 nm or less, still more preferably 180 nm or less. The ratio of constriction diameter/minor axis diameter is more preferably 0.80 or less, still more preferably 0.60 or less. The lower limit of the constriction diameter/minor axis diameter ratio is not particularly limited, and presumed to be about 0.40.

The major axis diameter, minor axis diameter, and constriction diameter of the particles may be measured by the following method.

Approximately 300 particles are observed with a transmission electron microscope to measure the major axis diameters, minor axis diameters, and constriction diameters defined above, and the respective average values are calculated.

Further, the ratio of constriction diameter/minor axis diameter of particles may be obtained by the following method.

Approximately 300 particles are observed with a transmission electron microscope to calculate the values of the ratio of constriction diameter/minor axis diameter from the minor axis diameter and constriction diameter values defined above for each particle, and the ratio is obtained by averaging the values.

As described above, the titanium (IV) oxide powder of the present invention blended in a cosmetic allows the cosmetic to spread easily and to have soft focus properties and appropriate hiding power. The reason is presumed as follows, though not being clear. The particles constituting the titanium (IV) oxide powder of the present invention have a shape in which two particles with a particle size of 100 nm to 400 nm are connected, so that the optical properties as an aggregate are similar to those of particles with a particle size of 100 nm to 400 nm. However, since the number of times that an incident light passes through the particle surface decreases, so that scattering and reflection are suppressed. On the other hand, in the titanium (IV) oxide powder of the present invention, due to the shape of the particles constituting the powder, many spaces are left inside the powder, and the light that enters the powder is scattered at the interface between the space and the particle. It is presumed that these effects compete against each other, resulting in the soft focus properties and appropriate hiding power. Further, the powder of the present invention has a smaller specific surface area and smaller contact points between particles in comparison with an aggregate of particles having a particle size of 100 nm to 400 nm, so that it is presumed that the powder spreads easily.

The titanium (IV) oxide in the powder of the present invention is preferably rutile-type titanium (IV) oxide. Rutile-type titanium oxide has a weaker blue color and lower activity in comparison with anatase-type titanium oxide. Further, compared with titanium (IV) oxide in crystal phases other than rutile-type and anatase-type, better productivity and stability can be obtained. The crystal phase of titanium (IV) oxide may be determined by confirming the main peak of each crystal phase (for example, the diffraction intensity of the (110) plane of rutile-type titanium (IV) oxide) using an X-ray diffraction device.

Each particle constituting the titanium (IV) oxide powder of the present invention may be composed of fine rod-like particles with a long axis diameter of 30 nm or more and 350 nm or less and a short axis diameter of 3 nm or more and 12 nm or less, and the fine rod-like particles aggregate into a particle having a peanut-like twin shape. In the case where the particles having a peanut-like twin shape formed from aggregation of fine rod-like particles are blended into a cosmetic, the makeup durability of the cosmetic can be improved. Although the reason for this is not clear, it is presumed that in the case where each of the peanut-like twin shaped particles is formed from aggregation of rod-shaped particles, many fine voids are created on the surface of the particles, allowing excess sebum and sweat to be gradually absorbed into the fine voids to improve the makeup durability. The fine rod-like particles have a lower limit of the long axis of preferably 150 nm or more, more preferably 200 nm or more, and an upper limit of preferably 325 nm, more preferably 300 nm or less, and have a lower limit of the short axis of preferably 8 nm or more, and an upper limit of 11 nm or less.

The long axis diameter and short axis diameter of the fine rod-like particles are the respective average values of the long axis diameter and short axis diameter of about 300 fine rod-like particles observed with a transmission electron microscope.

Incidentally, particles having a peanut-like twin shape may be formed by firing particles having a form in which fine rod-like particles are aggregated into a peanut-like twin shape so as to fuse the fine rod-like particles with each other. The particles constituting the powder of the present invention include both of those in which fine rod-shaped particles aggregate (without fusing) to form an apparently single particle, and those in which fine rod-shaped particles are fused to form a single particle. In the former case, as described above, when blended into a cosmetic, the effect of improving makeup durability can be obtained. In the latter case, a cosmetic more excellent in hiding power can be obtained.

The titanium (IV) oxide powder of the present invention preferably has a total light transmittance of 45.0% or more and 60.0% or less, and a haze value of 90.0% or more. The powder having such a total light transmittance and haze value blended into a cosmetic has soft focus properties and appropriate hiding power, so that a natural bare skin feel can be exhibited. Preferably, the total light transmittance has a lower limit of 50.0% or more and an upper limit of 60.0% or less, and the haze value has a lower limit of 91.0% or more. The upper limit of the haze value is not particularly limited, and may be 100.0%. The measurement method of total light transmittance and haze value is as follows.

The powder is added to a 1:1 mixture of trimethylsiloxysilicic acid and cyclopentane siloxane and stirred at 2000 rpm to prepare a coating material at a powder concentration of 100 g/kg. The coating material is formed into a coating film with a bar coater having a film thickness of 18.4 µm, and the total light transmittance and haze value of the coating film are measured with irradiation of C-light, which is light having a color temperature of 6774 K, by a double beam method.

The entire surface or at least a portion of the surface of the particles constituting the powder of the present invention may be coated with a layer of inorganic and/or organic substance. Examples of the inorganic substance may include one or more of aluminum, silicon, zinc, titanium, zirconium, iron, cerium, and tin, which are preferably an oxide, hydroxide, or oxyhydroxide thereof. Preferred examples of the organic substance include one or more of silicone-based compounds such as dimethylpolysiloxane and methyl hydrogen polysiloxane; coupling agents such as silane-based, aluminum-based, titanium-based and zirconium-based coupling agents; fluorine compounds such as perfluoroalkyl phosphoric acid compounds; and fatty acids including lauric acid and stearic acid. In addition, hydrocarbons, lecithin, amino acids, polyethylene, wax, etc. may also be used.

The titanium (IV) oxide powder of the present invention may be blended in cosmetics. The amount of titanium (IV) oxide powder blended in cosmetics varies depending on the form of the cosmetics, and in general, being preferably 1 g/kg or more and 500 g/kg or less, more preferably 10 g/kg or more and 450 g/kg or less.

### (Production method)

The titanium (IV) oxide particles having a peanut-like twin shape constituting the powder of the present invention may be obtained by a method including step 1 of adding hydrochloric acid to a slurry containing an acid-soluble titanium compound, and holding the mixture with a hydrochloric acid concentration of 60 g/L or more and 130 g/L or less at a temperature of 25°C or more and 60°C or less, for 1 hour or more to perform thermal hydrolysis, and a step 2 of holding the mixture with the hydrochloric acid concentration and/or the temperature higher than those in step 1 for 1 hour or more to perform thermal hydrolysis.

The acid-soluble titanium compound for use in the present invention may be any titanium compound soluble in hydrochloric acid, such as titanium hydroxide and sodium titanate. Among the acid-soluble titanium compounds, sodium titanate is particularly preferred.

In the hydrolysis reaction in step 1, as described above, the hydrochloric acid concentration is controlled and maintained at 60 g/L or more and 130 g/L or less, and the temperature is controlled and maintained at 25°C or more and 60°C or less. The lower limit of the hydrochloric acid concentration is more preferably 70 g/L or more, still more preferably 80 g/L or more. The lower limit of the temperature is more preferably 30°C or more, still more preferably 35°C or more, and the upper limit is more preferably 50°C or less, even more preferably 45°C or less. The holding time is not particularly limited as long as it is one hour or more, and from the viewpoint of energy efficiency, a short holding time is preferred. The holding time is preferably one hour or more and ten hours or less, and more preferably two hours or more and five hours or less.

In the hydrolysis reaction in step 2, at least one of the hydrochloric acid concentration and the temperature is made higher than in step 1. In step 2, it is more preferable to control both the hydrochloric acid concentration and the temperature higher than those in step 1. Further, in step 2, the upper limit of the hydrochloric acid concentration is controlled to 180 g/L or less, and the upper limit of the temperature is controlled to 100°C or less. The upper limit of the hydrochloric acid concentration is preferably 170 g/L or less, more preferably 160 g/L or less, and the temperature is preferably 70°C or less, still more preferably 65°C or less. The lower limits of both the hydrochloric acid concentration and the temperature are not particularly limited, but is preferably 60 g/L or more and 25°C or more, respectively, since it is preferred not to be less than step 1. The holding time in step 2 is not particularly limited as long as it is 1 hour or more, being preferably 1 hour or more and 30 hours or less, and more preferably 10 hours or more and 20 hours or less.

The hydrolysis reaction may be performed three or more times. The conditions for the third times and later are not particularly limited, and it is desirable that the hydrolysis reaction is performed under conditions similar to those for the first and second times, considering the equipment necessary for the hydrolysis.

It is desirable that the sample after hydrolysis is collected through holding the solution at a constant temperature to precipitate titanium (IV) oxide, and then through cooling, filtering, and washing the precipitate. Although the conditions for precipitation are not limited, it is desirable to maintain the temperature at 60°C or more and 100°C or less for 1 hour or more. The upper limit of the holding time during precipitation is not particularly limited, being preferably 8 hours or less. The cooling, filtration and washing methods are not particularly limited. It is preferable that conventional procedures in the field be employed.

The titanium (IV) oxide obtained through the steps 1 and 2 may be further fired (step 3). The titanium (IV) oxide particles having a peanut-like twin shape of the present invention may be fired to further improve the hiding power of a cosmetic when blended into the cosmetic, as needed. The firing temperature is preferably 700°C or less. In the case where the firing is performed at a temperature more than 700°C, sintering proceeds excessively, so that the particles having the peanut-like twin shape in the scope of the present invention may not be obtained, and the natural-looking bare skin cannot be obtained when the particles are used in a cosmetic. The lower limit of the firing temperature is not particularly limited. In one embodiment, it is preferably 300°C or more. In the case where the firing temperature is less than 300°C, the change in performance is small similarly to the case where no firing is performed, so that the value of firing is small from an industrial perspective. The holding time is preferably 10 minutes or more and 60 minutes or less. With a holding time of more than 60 minutes, sintering may proceed excessively and the particles having the peanut-like twin shape in the scope of the present invention may not be obtained. With a holding time of less than 10 minutes, uneven heat distribution may occur within the powder to cause non-uniform quality, which is industrially undesirable. The holding time is more preferably 20 minutes or more and 50 minutes or less.

The resulting titanium (IV) oxide particles (including fired products and non-fired products) may have at least a portion of the particle surface coated with an inorganic and/or organic layer. The method of applying the coating is not particularly limited, and conventional methods may be used. Coating at least a portion of the particle surface with an inorganic layer allows, for example, dispersion stability and durability in the dispersion medium to be improved. Examples of the inorganic substance that may be used include one or more metals such as aluminum, silicon, zinc, titanium, zirconium, iron, cerium, and tin. Oxides, hydroxides or oxyhydroxides thereof are preferred. There are no restrictions on the type of a salt of the metal.

Further, depending on the intended use of the cosmetic, at least a portion of the particle surface may be coated with a layer of an organic substance, to perform water and/or oil repellent treatment in the particles. Examples of the organic substance include silicone-based compounds such as dimethylpolysiloxane and methyl hydrogen polysiloxane; coupling agents such as silane-based, aluminum-based, titanium-based, and zirconium-based coupling agents; fluorine compounds such as perfluoroalkyl phosphoric acid compounds; hydrocarbons; lecithin; amino acids; polyethylene; wax; and fatty acids such as lauric acid and stearic acid.

### (Application)

The application of the titanium (IV) oxide powder of the present invention is not particularly limited, being particularly suitable for blending into cosmetics. The powder of the present invention allows cosmetics to be imparted with properties such as soft focus properties, appropriate hiding power, and non-stickiness.

In the case of using the titanium (IV) oxide powder of the present invention in cosmetics, the powder may be mixed with other cosmetic raw materials according to known methods. It is preferable that the surface of the powder be coated in advance as described above. In mixing with other cosmetic raw materials, the powder may be directly mixed with the other raw materials, or the powder may be dispersed in a dispersion medium to prepare a dispersion, and then the dispersion may be mixed with other raw materials. In the present invention, the term "cosmetics containing titanium (IV) oxide powder" refers to not only cosmetics containing titanium (IV) oxide powder as it is (in powder form), but also cosmetics containing the powder dispersed in a dispersion medium to form a dispersion and then blended into the cosmetics. The content of the titanium (IV) oxide powder of the present invention in cosmetics may be optionally set depending on the required characteristics of various cosmetics, being preferably 1 g/kg or more and 500 g/kg or less, more preferably 10 g/kg or more and 450 g/kg or less. A mixture of two or more types of titanium (IV) oxide powder composed of particles having a peanut-like twin shape according to the present invention, with one or more of the major axis diameter, minor axis diameter, and constriction diameter being different, may be used. Alternatively, into a cosmetic containing the powder of the present invention, titanium oxide having a different particle size and/or shape may be further blended depending on the purpose.

In preparation of a cosmetic containing the titanium (IV) oxide powder of the present invention, various components such as inorganic pigments and organic pigments used in ordinary cosmetics may be used in combination on an as needed basis. Examples of the inorganic pigments usable in combination include titanium oxide, zinc oxide, red iron oxide, yellow iron oxide, black iron oxide, brown iron oxide, ultramarine, Prussian blue, cerium oxide, talc, muscovite, synthetic mica, phlogopite, biotite, synthetic fluorophlogopite, mica titanium, micaceous iron oxide, sericite, zeolite, kaolin, bentonite, clay, silicic acid, silicic anhydride, magnesium silicate, aluminum silicate, calcium silicate, barium sulfate, magnesium sulfate, calcium sulfate, calcium carbonate, magnesium carbonate, boron nitride, bismuth oxychloride, alumina, zirconium oxide, magnesium oxide, chromium oxide, calamine, hydroxyapatite, and complexes thereof. Examples of the organic pigments usable in combination include silicone powder, silicone elastic powder, polyurethane powder, cellulose powder, nylon powder, urethane powder, silk powder, polymethyl methacrylate (PMMA) powder, starch, polyethylene powder, polystyrene powder, carbon black, tar pigments, natural pigments, metal soaps such as zinc stearate, and complexes thereof.

Cosmetics containing the titanium (IV) oxide powder of the present invention may contain other components in addition to the components described above depending on the purpose, within a quantitative and qualitative range that does not impair the effects of the present invention. For example, oily components, pigments, pH adjusters, moisturizers, thickeners, surfactants, dispersants, stabilizers, colorants, preservatives, antioxidants, sequestering agent, astringents, anti-inflammatory agents, ultraviolet absorber, fragrances, etc. in an appropriate amount may be blended within a range not impairing the effects of the present invention.

A cosmetic containing the titanium (IV) oxide powder of the present invention may be produced by a known method. The cosmetic may be in any dosage form such as powder, powder solid, cream, emulsion, lotion, oily liquid, oily solid, and paste. For example, makeup cosmetics, skin care cosmetics, and hair care cosmetics, which include makeup bases, foundations, concealers, face powder, control colors, sunscreen cosmetics, lipsticks, lip balms, eye shadows, eyeliners, mascaras, cheek colors, nail polishes, body powder, perfume powder, baby powder, etc. may be produced.

The titanium (IV) oxide powder of the present invention may be used in various fields where titanium oxide is generally used, in addition to cosmetics. Specific examples of the fields include resin compositions, coating materials, inks, external additives to toner, photocatalysts, electrical/electronic materials, ultraviolet blocking materials, heat insulating materials, radio wave blocking materials and ultraviolet blocking materials, though not limited thereto.

For example, the titanium (IV) oxide powder of the present invention has properties that a space between the particles is easily formed due to the particle shape, and accordingly, in the case where the powder is added to a resin, it is considered that an effect of preventing a formation of lumps can be obtained. It is also considered that in the case where the powder is used in an ink or coating material, fluidity can be maintained due to the small contact point between particles. In addition, due to the special shape, it is considered that the titanium (IV) oxide powder of the present invention has electrical and mechanical characteristics not found in conventional titanium (IV) oxide, and the novel characteristics may be applied to the field of electrical and electronic materials. That is, a cosmetic, a resin composition, a coating material, an ink, or an electrical/electronic material containing the titanium (IV) oxide powder of the present invention is an aspect of the present invention. From another point of view, the use of the titanium (IV) oxide powder of the present invention in cosmetics, resin compositions, coating materials, inks, or electrical/electronic materials is also an aspect of the present invention. Further, for example, the use of the titanium (IV) oxide powder of the present invention in production of cosmetics, resin compositions, coating materials, inks, or electrical/electronic materials can also be said to be one aspect of the present invention.

Prior to explaining Examples, the test methods used in the present invention are explained as follows.

### [Evaluation item and evaluation method]

### (Major axis diameter, minor axis diameter, constriction diameter and ratio of constriction diameter/minor axis diameter)

Measurement was performed using a transmission electron microscope JEM-1400plus manufactured by JEOL Ltd. The observation magnification was 50000 times (observation magnification power of transmission electron microscope: 10000, magnification power of print: 5). In each of the particles, a major axis diameter is the longest part, a minor axis diameter is the maximum part in the direction perpendicular to the major axis, and a constriction diameter is the length in the direction perpendicular to the major axis at the central position of the major axis. The major axis diameters, minor axis diameters, and constriction diameters of approximately 300 particles in the observation field were evaluated with an image analysis software ImageJ. The average values of the respective diameters of the about 300 particles were defined as the major axis diameter, minor axis diameter, and constriction diameter. Further, the value of constriction diameter/minor axis diameter was calculated by dividing the constriction diameter by the minor axis diameter for each of about 300 particles, and by calculating the average value thereof, the "Ratio of Constriction diameter/Minor axis diameter" was calculated.

### (Crystalline phase)

Using an X-ray diffractometer RINT-TTRIII manufactured by Rigaku Corporation under conditions with a target of copper (Cu), a tube voltage of 50 kV, a tube current of 300 mA, a divergent slit 1/2°, a divergent vertical slit of 10 mm, and a scattering slit of 1/2°, a light receiving slit of 0.15 mm and a scanning rate of 0.5°/min, a range from 20° to 35° of 20 was scanned to measure the diffraction intensity of (110) plane of rutile-type titanium dioxide (IV) and (101) plane of anatase-type titanium dioxide (IV).

### (Hiding ratio)

In a 140-mL mayonnaise bottle, 30.0 g of commercially available acrylic-based clear paint for automobile refinishing, 5.0 g of butyl acetate, and 10.0 g of titanium (IV) oxide powder were weighed and placed in DISPERMATFE/S manufactured by VMA-GETZMANN with an impeller having a diameter of 30 mm attached, so as to be dispersed at a rotation speed of 5000 rpm for 20 minutes, and then a dispersion was obtained. The dispersion in amount of 0.5 g was collected and dispersed by a Hoover Muller with 50 rotations under a load of 150 Lbs. The resulting dispersion was applied to a sheet of hiding ratio test paper Lot. NA220022 manufactured by Motofuji with a 3-mil applicator and then dried. The color tone was measured with an SM-7 type color computer manufactured by Suga Test Instruments Co., Ltd., and the ratio of L value on black background/L value on white background was defined as the hiding ratio.

### (Total light transmittance and haze value)

A sample in an amount of 1 g was added to trimethyl siloxy silicate KF-7312J manufactured by Shin-Etsu Chemical Co., Ltd. (mixture of trimethyl siloxy silicate and cyclopentane siloxane at a mass ratio of 1:1) in an amount of 9.0 times the mass, and the mixture was stirred with a Labolution (R) manufactured by PRIMIX Corporation at 2000 rpm to obtain a coating material having a powder concentration of 100 g/kg. The coating material was formed into a coating film using a bar coater with a film thickness of 18.4 µm. The resulting coating film was air-dried with cold air from a dryer until the surface was dried. The diffuse transmittance and total light transmittance were measured by a haze meter HZ-V3 manufactured by Suga Test Instruments Co., Ltd., irradiating C-light, which is light having a color temperature of 6774 K, by a double beam method. The haze value was calculated from (Diffuse transmittance/Total light transmittance)×100.

### EXAMPLES

The present invention is explained in more detail with reference to the following Examples. The Examples are given for illustrative purposes only and the scope of the invention is not limited thereto.

Incidentally, in the stirring operations described in Examples and Comparative Examples, the rotation speed is adjusted appropriately such that the entire liquid is mixed uniformly and droplets are prevented from scattering around, considering properties related to the behavior of the liquid during stirring such as the liquid volume, liquid viscosity, and container shape. In the case where the same effect can be obtained from a commercially available general product manufactured by any company such as sodium hydroxide, the name of the manufacturers and distributors are omitted.

### [Example 1]

Commercially available sodium titanate was dissolved in a mixture of water and 400 g/L hydrochloric acid to prepare a sodium titanate slurry with a TiO₂ concentration of 80 g/L and a hydrochloric acid concentration of 130 g/L. Subsequently, the slurry was heated while stirring using HEIDON (R) 600G manufactured by SHINTO Scientific Co., Ltd. (hereinafter referred to as the "stirrer"), and the liquid temperature was kept at 40°C for 3 hours, so that a first hydrolysis was performed. Subsequently, 400 g/L hydrochloric acid was added to the solution, and while adjusting the hydrochloric acid concentration to 150 g/L, the liquid was heated and held at a liquid temperature 45°C for 17 hours while stirring, so that a second hydrolysis was performed. Then, the liquid temperature was raised and kept at 80°C for 8 hours, so that titanium (IV) oxide was precipitated, and the precipitate was subjected to cooling, filtering, and washing to obtain a white powder.

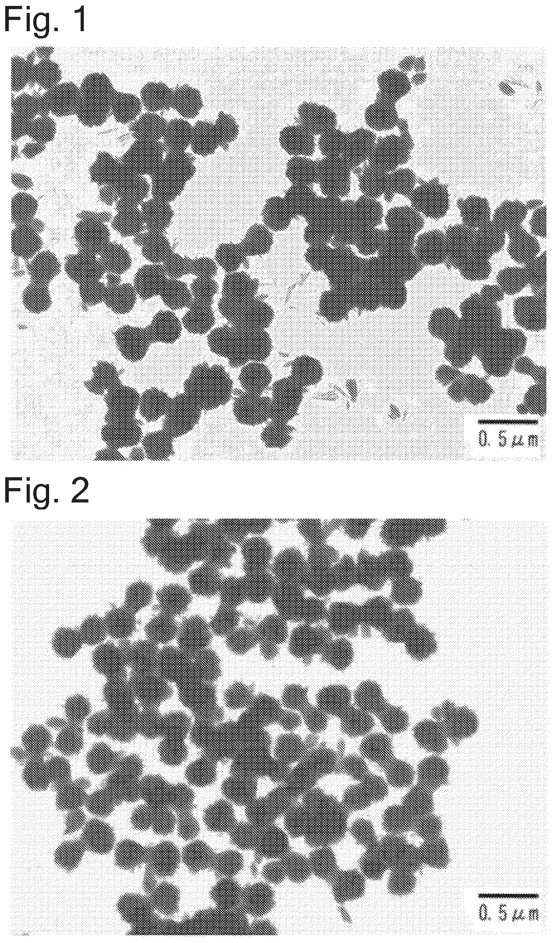

Fig. 1 is a transmission electron micrograph showing the resulting titanium (IV) oxide powder. The resulting titanium (IV) oxide powder was composed of peanut-like twin shape particles that are formed of aggregated fine rod-like particles with a long axis of 280 nm and a short axis of 10 nm, and that have a major axis diameter 560 nm, a minor axis diameter of 310 nm, a constriction diameter of 170 nm, and a constriction diameter/minor axis diameter ratio of 0.55, when measured by the method described above. The main peak was derived from rutile-type titanium (IV) oxide, when crystal phase was checked by the X-ray diffraction device. The hiding ratio was 0.83, total light transmittance was 52.0%, the diffuse transmittance was 48.4%, and haze value was 93.0%.

### [Example 2]

A white powder of titanium (IV) oxide was obtained in the same manner as in Example 1, except that the hydrochloric acid concentration was adjusted to 120 g/L in the first hydrolysis. Fig. 2 is a transmission electron micrograph showing the resulting titanium (IV) oxide powder. The resulting titanium (IV) oxide powder was composed of peanut-like twin shape particles that are formed of aggregated fine rod-like particles with a long axis of 265 nm and a short axis of 10 nm, and that have a major axis diameter 530 nm, a minor axis diameter of 260 nm, a constriction diameter of 150 nm, and a constriction diameter/minor axis diameter ratio of 0.58. The main peak was derived from rutile-type titanium (IV) oxide. Measured values of the hiding ratio, total light transmittance, diffuse transmittance, and haze value are shown in Table 3.

### [Example 3]

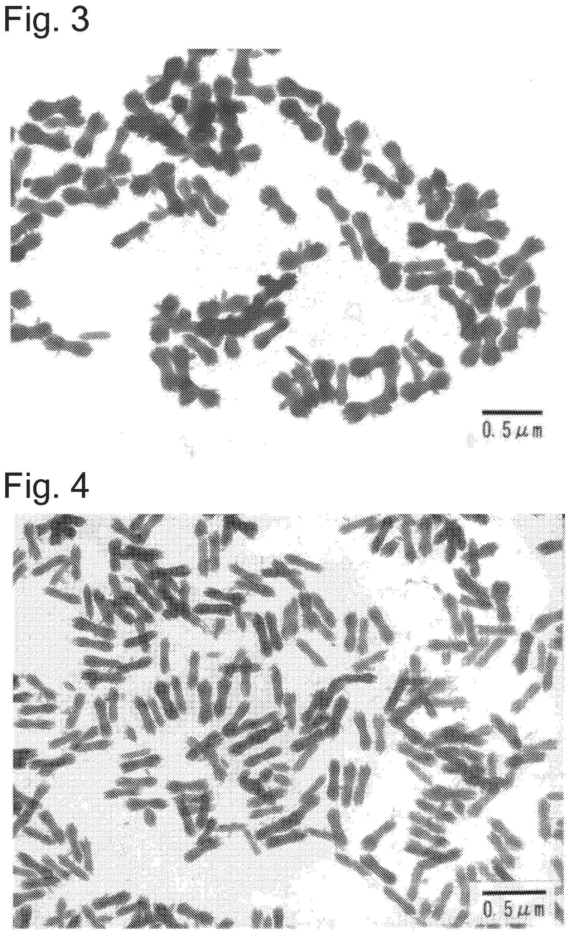

A white powder of titanium (IV) oxide was obtained in the same manner as in Example 1, except that the hydrochloric acid concentration was adjusted to 110 g/L in the first hydrolysis. Fig. 3 is a transmission electron micrograph showing the resulting titanium (IV) oxide powder. The resulting titanium (IV) oxide powder was composed of peanut-like twin shape particles that are formed of aggregated fine rod-like particles with a long axis of 210 nm and a short axis of 10 nm, and that have a major axis diameter 420 nm, a minor axis diameter of 180 nm, a constriction diameter of 110 nm, and a constriction diameter/minor axis diameter ratio of 0.61. The main peak was derived from rutile-type titanium (IV) oxide. Measured values of the hiding ratio, total light transmittance, diffuse transmittance, and haze value are shown in Table 3.

### [Example 4]

A white powder of titanium (IV) oxide was obtained in the same manner as in Example 1, except that the hydrochloric acid concentration was adjusted to 80 g/L in the first hydrolysis. Fig. 4 is a transmission electron micrograph showing the resulting titanium (IV) oxide powder. The resulting titanium (IV) oxide powder was composed of peanut-like twin shape particles that are formed of aggregated fine rod-like particles with a long axis of 185 nm and a short axis of 10 nm, and that have a major axis diameter 370 nm, a minor axis diameter of 110 nm, a constriction diameter of 85 nm, and a constriction diameter/minor axis diameter ratio of 0.77. The main peak was derived from rutile-type titanium (IV) oxide. Measured values of the hiding ratio, total light transmittance, diffuse transmittance, and haze value are shown in Table 3.

### [Example 5]

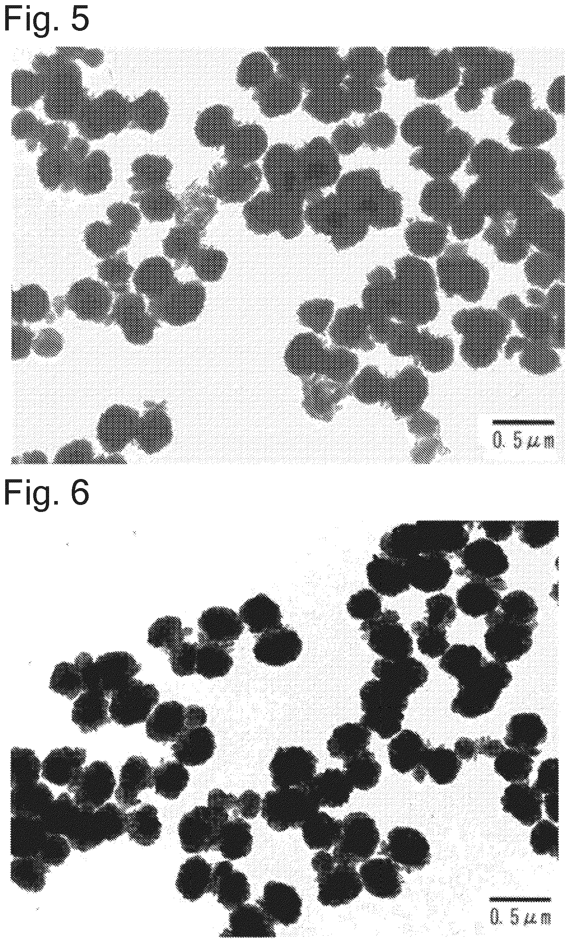

A white powder of titanium (IV) oxide was obtained in the same manner as in Example 1, except that the hydrochloric acid concentration was adjusted to 160 g/L in the second hydrolysis. Fig. 5 is a transmission electron micrograph showing the resulting titanium (IV) oxide powder. The resulting titanium (IV) oxide powder was composed of peanut-like twin shape particles that are formed of aggregated fine rod-like particles with a long axis of 320 nm and a short axis of 10 nm, and that have a major axis diameter 640 nm, a minor axis diameter of 360 nm, a constriction diameter of 180 nm, and a constriction diameter/minor axis diameter ratio of 0.50. The main peak was derived from rutile-type titanium (IV) oxide. Measured values of the hiding ratio, total light transmittance, diffuse transmittance, and haze value are shown in Table 3.

### [Example 6]

The titanium (IV) oxide powder obtained in Example 5 was fired at 650°C for 30 minutes with SUPER-C C-2035D manufactured by Motoyama Co., Ltd., and cooled to room temperature to obtain a white powder. Fig. 6 is a transmission electron micrograph showing the resulting titanium (IV) oxide powder. The resulting titanium (IV) oxide powder was composed of particles having a peanut-like twin shape, with a major axis diameter 610 nm, a minor axis diameter of 355 nm, a constriction diameter of 195 nm, and a constriction diameter/minor axis diameter ratio of 0.55. The fine rod-like particles observed in Examples 1 to 5 were not observed. The main peak was derived from rutile-type titanium (IV) oxide. Measured values of the hiding ratio, total light transmittance, diffuse transmittance, and haze value are shown in Table 3.

### [Comparative Example 1]

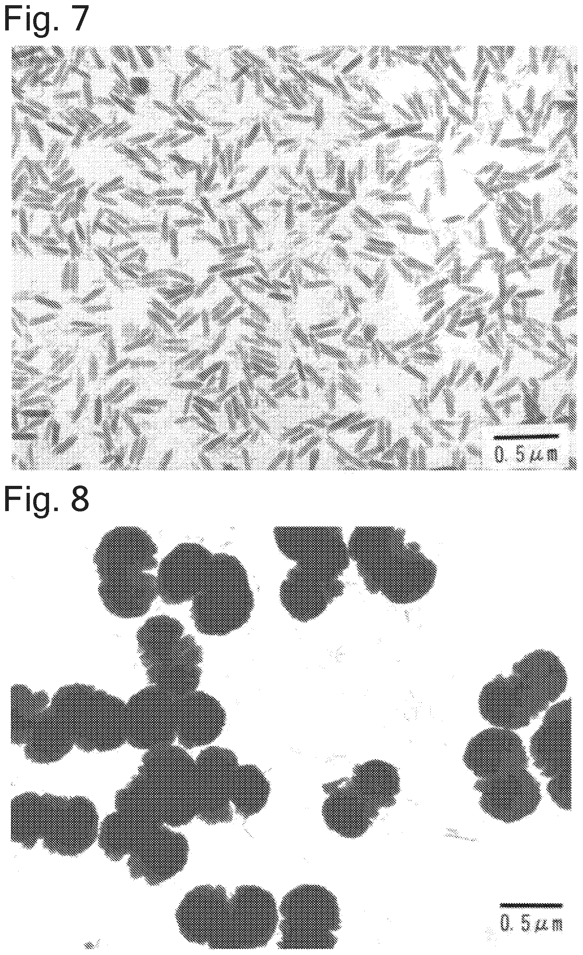

A white powder of titanium (IV) oxide was obtained in the same manner as in Example 1, except that the hydrochloric acid concentration was adjusted to 50 g/L in the first hydrolysis. Fig. 7 is a transmission electron micrograph showing the resulting titanium (IV) oxide powder. The resulting titanium (IV) oxide powder was composed of strip shape particles that are formed of aggregated fine rod-like particles with a long axis of 120 nm and a short axis of 10 nm, and that have a major axis diameter 240 nm, a minor axis diameter of 55 nm, a constriction diameter of 50 nm, and a constriction diameter/minor axis diameter ratio of 0.91. The main peak was derived from rutile-type titanium (IV) oxide. Measured values of the hiding ratio, total light transmittance, diffuse transmittance, and haze value are shown in Table 3.

### [Comparative Example 2]

A white powder of titanium (IV) oxide was obtained in the same manner as in Example 5, except that the hydrochloric acid concentration was adjusted to 140 g/L in the first hydrolysis. Fig. 8 is a transmission electron micrograph showing the resulting titanium (IV) oxide powder. The resulting titanium (IV) oxide powder had agglomerated particles without constriction, which are formed of aggregated fine rod-like particles with a long axis of 410 nm and a short axis of 10 nm, and which have a major axis diameter of 820 nm, a minor axis diameter of 490 nm, a constriction diameter (diameter perpendicular to the long axis at the center of the long axis) of 540 nm, and a constriction diameter/minor axis diameter ratio of 1.10. The main peak was derived from rutile-type titanium (IV) oxide. Measured values of the hiding ratio, total light transmittance, diffuse transmittance, and haze value are shown in Table 3.

### [Comparative Example 3]

A commercially available rutile-type titanium (IV) oxide powder for use in cosmetics was used. The titanium oxide powder is formed of non-uniform shape particles, having a major axis diameter 340 nm and a minor axis diameter of 240 nm, a constriction diameter of 240 nm, and a constriction diameter/minor axis diameter ratio of 1.00. Measured values of the hiding ratio, total light transmittance, diffuse transmittance, and haze value are shown in Table 3.

In Table 1, production conditions for the titanium (IV) oxide powders in Examples and Comparative Examples are shown, in Table 2, the characteristics of particles constituting the titanium (IV) oxide powders obtained in Examples and Comparative Examples are shown, and in Table 3, the characteristics of the titanium (IV) oxide powders in Examples and Comparative Examples are shown.

**[Table 1]**

| | TiO₂ concentration | Hydrolysis | | | | | | Maturing | | Firing | |
|---|---|---|---|---|---|---|---|---|---|---|---|
| | | First time | | | Second time | | | | | | |
| | | Hydrochloric acid concentration | Temperature | Holding time | Hydrochloric acid concentration | Temperature | Holding time | Temperature | Holding time | Temperature | Holding time |
| | g/L | g/L | °C | Hour | g/L | °C | Hour | °C | Hour | °C | Minute |
| Example 1 | 80 | 130 | 40 | 3 | 150 | 45 | 17 | 80 | 8 | - | - |
| Example 2 | 80 | 120 | 40 | 3 | 150 | 45 | 17 | 80 | 8 | - | - |
| Example 3 | 80 | 110 | 40 | 3 | 150 | 45 | 17 | 80 | 8 | - | - |
| Example 4 | 80 | 80 | 40 | 3 | 150 | 45 | 17 | 80 | 8 | - | - |
| Example 5 | 80 | 130 | 40 | 3 | 160 | 45 | 17 | 80 | 8 | - | - |
| Example 6 | 80 | 130 | 40 | 3 | 160 | 45 | 17 | 80 | 8 | 650 | 30 |
| Comparative Example 1 | 80 | 50 | 40 | 3 | 150 | 45 | 17 | 80 | 8 | - | - |
| Comparative Example 2 | 80 | 140 | 40 | 3 | 160 | 45 | 17 | 80 | 8 | - | - |
| Comparative Example 3 | - | - | - | - | - | - | - | - | - | - | - |

**[Table 2]**

| | Shape | Particle diameter | | | Ratio of Constriction diameter/Minor axis diameter |
|---|---|---|---|---|---|
| | | Major axis | Minor axis | Constriction | |
| | | nm | | | - |
| Example 1 | Peanut-like twin shape | 560 | 310 | 170 | 0.55 |
| Example 2 | Peanut-like twin shape | 530 | 260 | 150 | 0.58 |
| Example 3 | Peanut-like twin shape | 420 | 180 | 110 | 0.61 |
| Example 4 | Peanut-like twin shape | 370 | 110 | 85 | 0.77 |
| Example 5 | Peanut-like twin shape | 640 | 360 | 180 | 0.50 |
| Example 6 | Peanut-like twin shape | 610 | 355 | 195 | 0.55 |
| Comparative Example 1 | Strip shape | 240 | 55 | 50 | 0.91 |
| Comparative Example 2 | Agglomerate without constriction | 820 | 490 | 540 | 1.10 |
| Comparative Example 3 | Non-uniform shape | 340 | 240 | 240 | 1.00 |

**[Table 3]**

| | Hiding ratio | Total light transmittance | Diffuse transmittance | Haze value |
|---|---|---|---|---|
| | - | % | % | % |
| Example 1 | 0.83 | 52.0 | 48.4 | 93.0 |
| Example 2 | 0.83 | 51.7 | 48.6 | 93.9 |
| Example 3 | 0.82 | 51.5 | 47.0 | 91.3 |
| Example 4 | 0.80 | 51.2 | 46.1 | 90.0 |
| Example 5 | 0.81 | 59.1 | 54.5 | 92.2 |
| Example 6 | 0.90 | 52.2 | 47.9 | 91.7 |
| Comparative Example 1 | 0.74 | 65.2 | 53.6 | 82.2 |
| Comparative Example 2 | 0.76 | 61.6 | 57.1 | 92.7 |
| Comparative Example 3 | 0.93 | 40.7 | 38.3 | 94.3 |

As is clear from Table 3, the hiding ratios of the titanium (IV) oxide powders obtained in Examples 1 to 6 are greater than those in Comparative Examples 1 and 2. It is predicted that the titanium (IV) oxide powders of Examples 1 to 6 blended into cosmetics can impart appropriate covering power to the cosmetics. Further, the powders obtained in Examples 1 to 6 have a haze value as large as that of the commercially available titanium oxide in Comparative Example 3, and have a higher total light transmittance than that in Comparative Example 3, so that the cosmetics containing any of the powders in Examples 1 to 6 are expected to have excellent soft focus properties in addition to the covering power.

### (Cosmetics)

Hereinafter, cosmetics containing the titanium (IV) oxide powder of the present invention are explained.

### [Formulation example 1: Powder foundation]

Powder foundations were prepared by blending the powders (titanium oxide samples) obtained in Examples and Comparative Examples.

| (Components) | Blendiing ratio (g/kg) |
|---|---|
| 1. POE modified silicone (HLB=4.5) | 8 |
| 2. Polyglyceryl polyricinoleate | 5 |
| 3. Neopentyl glycol dicaprate | 30 |
| 4. Squalane | 10 |
| 5. Pentaerythrityl tetraoctanoate | 20 |
| 6. Inulin stearate (Note 1) | 10 |
| 7. Cyclomethicone | 94 |
| 8. Preservative | appropriate amount |
| 9. Antioxidant | appropriate amount |
| 10. Fragrance | appropriate amount |
| 11. Silicone treated titanium oxide sample (Note 2) | 100 |
| 12. Silicone treated talc (Note 2) | 40 |
| 13. Silicone treated colorant (Note 2) | 10 |
| 14. Purified water | balance |
| 15. 1,3-Butylene glycol | 50 |
| 16. Glycerol | 10 |
| 17. Sodium chloride | 10 |
| 18. Preservative | appropriate amount |

| | |
|---|---|
| (Note 1) Rheopearl (R) ISK, manufactured by Chiba Flour Milling Co., Ltd. (Note 2) Surface treated with KF-9909 manufactured by Shin-Etsu Chemical Co., Ltd. | |

### (Production method)

A: Components 11 to 13 were stirred and mixed with LAB. MIXER LM-110T manufactured by HANII, Electric. Co., Lid (hereinafter referred to as "Henschel mixer").
B: A was added to components 1 to 10 and uniformly dispersed using a Henschel mixer.
C: Components 14 to 18 were heated and dissolved in a separate container.
D: C was added to B and emulsified. The emulsion was then cooled to room temperature to obtain a powder foundation.

### (Sensory evaluation)

The resulting powder foundation was subjected to sensory tests for spreadability, smoothness, covering power, white cast, and makeup durability.

### [Evaluation and evaluation criteria]

Powder foundations prepared from the powders in Examples 1 to 6 and Comparative Examples 1 to 3 were used by 10 panelists, and the sensory evaluation items in Table 4 were evaluated on a scale of 1 to 5 and determined based on the average score.

### (Evaluation criteria)

Very good: 5 points, Good: 4 points, Fair: 3 points, Somewhat poor: 2 points, Poor: 1 point

### (Determination criteria)

Average score of 4.0 or more and 5.0 or less: A
Average score of 3.0 or more and less than 4.0: B
Average score of 2.0 or more and less than 3.0: C
Average score of 1.0 or more and less than 2.0: D

The results are shown in Table 4.

**[Table 4]**

| | Spreadability | Smoothness | Covering power | White cast | Makeup durability |
|---|---|---|---|---|---|
| Example 1 | A | A | A | B | B |
| Example 2 | A | B | B | B | B |
| Example 3 | B | B | B | B | B |
| Example 4 | B | B | C | A | B |
| Example 5 | A | A | B | B | B |
| Example 6 | A | A | A | B | B |
| Comparative Example 1 | C | C | C | A | C |
| Comparative Example 2 | B | B | C | B | B. |
| Comparative Example 3 | D | D | A | D | C |

As a result of the sensory test, the powder foundations containing the powders obtained in Examples 1 to 6 were excellent in spreadability, smoothness, covering power, and makeup durability, and all of them had rare occurrence of white cast. The titanium (IV) oxide powder of the present invention composed of particles having a peanut-like twin shape blended into a cosmetic imparted easy spreadability, soft focus properties, and appropriate hiding power to the cosmetic. As a result, a cosmetic that has an excellent cosmetic film uniformity, blurs age spots and pores, and exhibits a natural-looking bare skin feel was provided.

### [Formulation example 2: W/O emulsion-type foundation]

A W/O emulsion-type foundation was prepared using the powder obtained in Example.

| (Components) | Blending ratio (g/kg) |
|---|---|
| 1. Crosslinked polyether-modified silicone (Note 1) | 20 |
| 2. Crosslinked dimethylpolysiloxane (Note 2) | 30 |
| 3. Decamethyl cyclopentasiloxane | 135 |
| 4. Dimethylpolysiloxane (6 mm²/s (25°C)) | 70 |
| 5. Silicone-treated powder in Example 1 (Note 3) | 250 |
| 6. Silicone treated talc (Note 3) | 40 |
| 7. 1,3-butylene glycol | 50 |
| 8. Sodium citrate | 4 |
| 9. sodium chloride | 5 |
| 10. Preservative | appropriate amount |
| 11. Purified water | balance |

| | |
|---|---|
| (Note 1) KSG-210, manufactured by Shin-Etsu Chemical Co., Ltd. (Note 2) KSG-15, manufactured by Shin-Etsu Chemical Co., Ltd. (Note 3) Surface treated with KF-9909 manufactured by Shin-Etsu Chemical Co., Ltd. | |

### (Production method)

A: Components 1 to 5 were uniformly mixed with Labolution (R) manufactured by PRIMIX Corporation (hereinafter referred to as "mixer"), and further, component 6 was uniformly dispersed with a Henschel mixer.
B: Components 7 to 11 were uniformly mixed with a mixer.
C: While stirring, B was gradually added to A to be emulsified, so that a W/O emulsion-type foundation was obtained.

The resulting W/O emulsion-type foundation was excellent in spreadability, and after application, due to the soft focus properties and appropriate hiding power, no white cast occurred, with a finish blurring age spots and pores to exhibit a natural-looking bare skin feel.

### [Formulation example 3: W/O cream]

A W/O cream was prepared using the powder obtained in Example.

| (Components) | Blending ratio (g/kg) |
|---|---|
| 1. Crosslinked polyether-modified silicone (Note 1) | 35 |
| 2. Crosslinked dimethylpolysiloxane (Note 2) | 50 |
| 3. Branched-type polyether modified silicone (Note 3) | 10 |
| 4. Organically modified bentonite | 12 |
| 5. Triethylhexanoin | 50 |
| 6. Dimethylpolysiloxane (6 mm²/s (25°C)) | 55 |
| 7. Decamethyl cyclopentasiloxane | 90 |
| 8. Dissolved acrylic silicone resin (Note 4) | 15 |
| 9. Silicone-treated powder in Example 2 (Note 5) | 250 |
| 10. 1,3-Butylene glycol | 50 |
| 11. Sodium citrate | 4 |
| 12. Purified water | balance |

| | |
|---|---|
| (Note 1) KSG-210, manufactured by Shin-Etsu Chemical Co., Ltd. (Note 2) KSG-15, manufactured by Shin-Etsu Chemical Co., Ltd. (Note 3) KF-6028, manufactured by Shin-Etsu Chemical Co., Ltd. (Note 4) KP-575, manufactured by Shin-Etsu Chemical Co., Ltd. (Note 5) Surface treated with KF-9909 manufactured by Shin-Etsu Chemical Co., Ltd. | |

### (Production method)

A: Components 1 to 8 were mixed and uniformly dispersed.
B: Components 10 to 12 were mixed and dissolved.
C: B was added to A and uniformly mixed.
D: Component 9 was added to C and uniformly mixed to obtain a W/O cream. The resulting W/O cream was easily spread without creaky feel, excellent in adhesion.

### [Formulation example 4: W/O emulsion-type sunscreen]

A W/O emulsion-type sunscreen was prepared using the powder obtained in Example.

| (Components) | Blending ratio (g/kg) |
|---|---|
| 1. Caprylyl silane treated mica (Note 1) | 400 |
| 2. Powder in Example 2 | 50 |
| 3. Silicone treated talc (Note 2) | balance |
| 4. Silicone treated pigment grade titanium oxide (Note 2) | 50 |
| 5. Silicone treated titanium oxide fine particle (Note 2) | 50 |
| 6. silicone treated barium sulfate (Note 2) | 100 |
| 7. Silicone treated red iron oxide (Note 2) | 4 |
| 8. Silicone treated yellow iron oxide (Note 2) | 20 |
| 9. Silicone treated amber (Note 2) | 4 |
| 10. Silicone treated black iron oxide (Note 2) | 1 |
| 11. Phenyl modified hybrid silicone composite powder (Note 3) | 20 |
| 12. Spherical polymethyl silsesquioxane powder (Note 4) | 5 |
| 13. Preservative | appropriate amount |
| 14. Fragrance | appropriate amount |
| 15. Crosslinked dimethylpolysiloxane (Note 5) | 40 |
| 16. Glyceryl trioctanoate | 20 |
| 17. Squalane | 10 |

| | |
|---|---|
| (Note 1) Surface treated with AES-3083, manufactured by Shin-Etsu Chemical Co., Ltd. (Note 2) Surface treated with KF-9909, manufactured by Shin-Etsu Chemical Co., Ltd. (Note 3) KSP-300, manufactured by Shin-Etsu Chemical Co., Ltd. (Note 4) KMP-590, manufactured by Shin-Etsu Chemical Co., Ltd. (Note 5) KSG-16, manufactured by Shin-Etsu Chemical Co., Ltd. | |

### (Production method)

A: Components 1 to 13 were mixed and uniformly pulverized.
B: Components 15 to 17 uniformly mixed with a mixer were added to A, and the whole was homogenized.
C: Component 14 was added to B, and the mixture was subjected to press molding in a mold to obtain a W/O emulsion-type sunscreen.

The resulting W/O emulsion sunscreen was very easily spread without stickiness, and excellent in adhesion.

### [Formulation example 5: Pressed powder foundation 1]

A pressed powder foundation was prepared using the powder obtained in Example.

| (Components) | Blending ratio (g/kg) |
|---|---|
| 1. Talc | balance |
| 2. PMMA (7 µm) (Note 1) | 100 |
| 3. Sericite | 300 |
| 4. Scaly silica (Note 2) | 30 |
| 5. Powder in Example 3 | 60 |
| 6. Preservative | appropriate amount |
| 7. Colorant | appropriate amount |
| 8. Ethylhexyl methoxycinnamate | 30 |
| 9. Squalane | 20 |
| 10. Preservative | appropriate amount |
| 11. Antioxidant | appropriate amount |
| 12. Fragrance | appropriate amount |

| | |
|---|---|
| (Note 1) Matsumoto Microsphere M-100, manufactured by Matsumoto Yushi-Seiyaku Co., Ltd. (Note 2) Sunlovely (R) C, manufactured by AGC Si-Tech Co., Ltd. | |

### (Production method)

A: Components 1 to 7 were mixed and pulverized.
B: A was transferred into a Henschel mixer, and components 8 to 12 were added thereto. The mixture was stirred and mixed to be homogenized.
C: B was pulverized with a sample mill TASM-1 manufactured by Tokyo Atomizer M.F.G. Co., Ltd. (hereinafter referred to as "atomizer"), and press-molded in an aluminum plate to obtain a pressed powder foundation.

The resulting pressed powder foundation was excellent in spreadability, and after application, due to the soft focus properties and appropriate covering power, no white cast occurred, with a finish blurring age spots and pores to exhibit a natural-looking bare skin feel.

### [Formulation example 6: Pressed powder foundation 2]

A pressed powder foundation was prepared using the powder obtained in Example.

| (Components) | Blending ratio (g/kg) |
|---|---|
| 1. Talc | balance |
| 2. Mica | 310 |
| 3. Sericite | 220 |
| 4. Powder in Example 3 | 88 |
| 5. Brown iron oxide (Note 1) | 13 |
| 6. Preservative | appropriate amount |
| 7. Dimethicone | 66 |
| 8. Neopentyl glycol diethylhexanoate | 12 |
| 9. Squalane | 36 |
| 10. Dipentaerythrite fatty acid ester (Note 2) | 6 |
| 11. Antioxidant | appropriate amount |
| 12. Fragrance | appropriate amount |

| | |
|---|---|
| (Note 1) BBR-213HP, manufactured by Titan Kogyo, Ltd. (Note 2) COSMOL (R) 168ARV, manufactured by The Nisshin OilliO Group Ltd. | |

### (Production method)

A: Components 1 to 6 were mixed and pulverized.
B: A was transferred into a Henschel mixer, and components 8 to 12 were added thereto. The mixture was stirred and mixed to be homogenized.
C: B was pulverized with an atomizer, and press-molded in an aluminum plate to obtain a pressed powder foundation.

The resulting pressed powder foundation was excellent in spreadability, and after application, due to the soft focus properties and appropriate covering power, no white cast occurred, with a finish blurring age spots and pores to exhibit a natural-looking bare skin feel.

### [Formulation example 7: 2-way Cake foundation]

A 2-way cake foundation was prepared using the powder obtained in Example.

| (Components) | Blending ratio (g/kg) |
|---|---|
| 1. Silicone treated talc (Note 1) | balance |
| 2. Powder in Example 4 | 100 |
| 3. Silicone treated mica (Note 1) | 200 |
| 4. silicone treated sericite (Note 1) | 360 |
| 5. Nylon powder | 100 |
| 6. Silicone treated yellow iron oxide (Note 1) | 10 |
| 7. Silicone treated red iron oxide (Note 1) | 5 |
| 8. Silicone treated black iron oxide (Note 1) | 1 |
| 9. Dimethylpolysiloxane 1000 cs | 60 |
| 10. Isotridecyl isononanoate | 30 |
| 11. Squalane | 30 |
| 12. Preservative | 2 |
| 13. Antioxidant | 1 |

| | |
|---|---|
| (Note 1) Surface treated with KF-99P, manufactured by Shin-Etsu Chemical Co., Ltd. | |

### (Production method)

A: Components 9 to 13 were heated and dissolved.
B: Components 1 to 8 were mixed with a Henschel mixer, and A was mixed thereinto.
C: B was pulverized with an atomizer, and press-molded in an aluminum plate to obtain a 2-way cake foundation.

The resulting 2-way cake foundation was excellent in spreadability, and after application, due to the soft focus properties and appropriate covering power, no white cast occurred, with a finish blurring age spots and pores to exhibit a natural-looking bare skin feel.

### [Formulation example 8: Oil-based cake foundation]

An oil-based cake foundation was prepared using the powder obtained in Example.

| (Components) | Blending ratio (g/kg) |
|---|---|
| 1. Silicone treated talc (Note 1) | 53 |
| 2. Powder in Example 5 | 150 |
| 3. Silicone treated sericite (Note 1) | 282 |
| 4. silicone treated red iron oxide (Note 1) | 5 |
| 5. Silicone treated yellow iron oxide (Note 1) | 18 |
| 6. Silicone treated black iron oxide (Note 1) | 2 |
| 7. Candelilla wax | 10 |
| 8. Carnauba wax | 10 |
| 9. Ceresin | 15 |
| 10. Decamethyl cyclopentasiloxane | 140 |
| 11. Isononyl isononanoate | balance |
| 12. Polyglyceryl diisostearate | 20 |
| 13. Dextrin palmitate | 10 |
| 14. Ethylhexyl methoxycinnamate | 30 |
| 15. Preservative | appropriate amount |
| 16. Antioxidant | appropriate amount |

| | |
|---|---|
| (Note 1) Surface treated with KF-9901, manufactured by Shin-Etsu Chemical Co., Ltd. | |

### (Production method)

A: Components 1 to 6 were mixed with a Henschel mixer, and the mixture was uniformly pulverized.
B: Components 7 to 16 were heated and dissolved, and A was mixed thereinto and uniformly stirred.
C: After defoaming, B was poured into a tray, and gradually cooled to room temperature, so that an oil-based cake foundation was obtained.

The resulting oil-based cake foundation was excellent in spreadability, and after application, due to the soft focus properties and appropriate covering power, no white cast occurred, with a finish blurring age spots and pores to exhibit a natural-looking bare skin feel.

### [Formulation example 9: Stick foundation]

A stick foundation was prepared using the powder obtained in Example.

| (Components) | Blending ratio (g/kg) |
|---|---|
| 1. Dimethylpolysiloxane | 180 |
| 2. Decamethyl cyclopentasiloxane | 300 |
| 3. Ethylhexyl methoxycinnamate | 50 |
| 4. Diisostearyl malate | 40 |
| 5. Candelilla wax | 60 |
| 6. Hydrogenated jojoba ester | 40 |
| 7. Cetyl dimethicone copolyol | 20 |
| 8. Sorbitan sesquiisostearate | 5 |
| 9. Antioxidant | appropriate amount |
| 10. Preservative | appropriate amount |
| 11. Fragrance | appropriate amount |
| 12. Silicone treated colorant (Note 1) | 5 |
| 13. Powder in Example 3 | 85 |
| 14. Silicone treated talc (Note 1) | 60 |
| 15. Silicone treated mica (Note 1) | 20 |
| 16. Polymethyl methacrylate | 20 |
| 17. Purified water | balance |
| 18. Sodium citrate | 3 |
| 19. 1,3-Butylene glycol | 30 |
| 20. Glycerol | 20 |
| 21. Preservative | appropriate amount |

| | |
|---|---|
| (Note 1) Surface treated with KF-99P, manufactured by Shin-Etsu Chemical Co., Ltd. | |

### (Production method)

A: Components 12 to 16 were mixed with a Henschel mixed.
B: Components 1 to 11 were weighed in a container capable of containing the entire amount, heated and dissolved.
C: Components 17 to 21 were weighed in another container, heated and dissolved.
D: A was added to B and uniformly dispersed. C was added thereto and emulsified.
E: After defoaming, D was poured into a mold, and gradually cooled to room temperature, so that a stick foundation was obtained.

The resulting stick foundation was excellent in spreadability, and after application, due to the soft focus properties and appropriate covering power, no white cast occurred, with a finish blurring age spots and pores to exhibit a natural-looking bare skin feel.

### [Formulation example 10: O/W emulsion-type foundation]

An O/W emulsion-type foundation was prepared using the powder obtained in Example.

| (Components) | Blending ratio (g/kg) |
|---|---|
| 1. Stearic acid | 4 |
| 2. Isostearic acid | 3 |
| 3. Cetyl 2-ethylhexanoate | 40 |
| 4. Liquid paraffin | 110 |
| 5. POE (10) stearyl ether | 20 |
| 6. Cetyl alcohol | 3 |
| 7. Preservative | 2 |
| 8. Talc | 150 |
| 9. Colorant | 40 |
| 10. Powder in Example 1 | 30 |
| 11. Triethanol amine | 4 |
| 12. Propylene glycol | 50 |
| 13. Purified water | 541 |
| 14. Preservative | 2 |
| 15. Antioxidant | 1 |

### (Production method)

A: Components 1 to 7 were heated and dissolved at 85°C.
B: Components 8 to 10 were mixed and pulverized.
C: Components 11 to 15 were heated at 85°C, dissolved and mixed.
D: B was added to A and uniformly dispersed. C was gradually added thereto and emulsified. The emulsion was cooled to room temperature while stirring. Subsequently, an appropriate container was filled with the emulsion, so that an O/W emulsion-type foundation was obtained.

The resulting O/W emulsion-type foundation was excellent in spreadability, and after application, due to the soft focus properties and appropriate covering power, no white cast occurred, with a finish blurring age spots and pores to exhibit a natural-looking bare skin feel.

### [Formulation example 11: Moisturizing O/W cream]

A moisturizing O/W cream was prepared using the powder obtained in Example.

| (Components) | Blending ratio (g/kg) |
|---|---|
| 1. Crosslinked dimethylpolysiloxane (Note 1) | 80 |
| 2. Crosslinked dimethylpolysiloxane (Note 2) | 280 |
| 3. Decamethyl cyclopentane siloxane | 100 |
| 4. Powder in Example 2 | 50 |
| 5. Branched polyglycerol modified silicone (Note 3) | 3 |
| 6. Branched polyglycerol modified silicone (Note 4) | 6 |
| 7. (Acrylamide/acryloyl dimethyl taurine Na) copolymer (Note 5) | 6 |
| 8. Dimethyl taurine ammonium acrylate/VP copolymer (5% aqueous solution) (Note 6) | 120 |
| 9. Polyethylene glycol 400 | 10 |
| 10. Sodium lactate | 50 |
| 11. 1,3-Butylene glycol | 50 |
| 12. Purified water | 245 |

| | |
|---|---|
| (Note 1) KSG-15, manufactured by Shin-Etsu Chemical Co., Ltd. (Note 2) KSG-16, manufactured by Shin-Etsu Chemical Co., Ltd. (Note 3) KF-6104, manufactured by Shin-Etsu Chemical Co., Ltd. (Note 4) KF-6100, manufactured by Shin-Etsu Chemical Co., Ltd. (Note 5) Simulgel 600, manufactured by Seppic S.A. (Note 6) Aristoflex (R) AVC, manufactured by Clariant Japan K.K. | |

### (Production method)

A: Components 3 to 5 were mixed and uniformly dispersed.
B: Components 1 and 2, and A were mixed, and the mixture was uniformly mixed with a mixer.
C: Components 6 to 12 were uniformly mixed with a mixer.
D: While stirring, B was gradually added to C to be emulsified, so that a moisturizing O/W cream was obtained.

The resulting moisturizing O/W cream was easily spread, and was smooth without stickiness or greasiness and creaky feel, with refreshing feel, and excellent in stability.

### [Formulation example 12: O/W cream]

An O/W cream was prepared using the powder obtained in Example.

| (Components) | Blending ratio (g/kg) |
|---|---|
| 1. Crosslinked dimethylpolysiloxane (Note 1) | 50 |
| 2. Powder in Example 1 | 10 |
| 3. Glyceryl triisostearate | 80 |
| 4. Cetanol | 5 |
| 5. Stearic acid | 10 |
| 6. Glyceryl monostearate | 5 |
| 7. Sorbitan sesquioleate | 5 |
| 8. Polyoxyethylene sorbitan monooleate | 10 |
| 9. Triethanol amine | 5 |
| 10. Carbomer (1% aqueous solution) | 197 |
| 11. Locust bean gum (2% aqueous solution) | 50 |
| 12. 1,3-Butylene glycol | 70 |
| 13. Preservative | appropriate amount |
| 14. Fragrance | appropriate amount |
| 15. Purified water | 500 |

| | |
|---|---|
| (Note 1) KSG-15, manufactured by Shin-Etsu Chemical Co., Ltd. | |

### (Production method)

A: Components 1 to 8 were heated and uniformly mixed.
B: Components 9 and 13, and 15 were mixed and heated.
C: While stirring, B was gradually added to A to be emulsified, and after cooling, component 14 was added thereto, so that an O/W cream was obtained.

The resulting O/W cream was easily spread, and was smooth without stickiness or greasiness and creaky feel, with refreshing feel, excellent in stability.

### [Formulation example 13: Body lotion]

A body lotion was prepared using the powder obtained in Example.

| (Components) | Blending ratio (g/kg) |
|---|---|
| 1. Ethanol | 170 |
| 2. 1,3-Butylene glycol | 30 |
| 3. Branched polyglycerol modified silicone (Note 1) | 5 |
| 4. Glyceryl trioctanoate | 10 |
| 5. powder in Example 4 | 20 |
| 6. Hybrid silicone composite powder (Note 2) | 100 |
| 7. Dimethyl taurine ammonium acrylate/VP copolymer | 4 |
| 8. Xanthan gum (2% aqueous solution) | 60 |
| 9. Sodium chloride | 1 |
| 10. Purified water | 600 |

| | |
|---|---|
| (Note 1) KF-6100, manufactured by Shin-Etsu Chemical Co., Ltd. (Note 2) KSP-100, manufactured by Shin-Etsu Chemical Co., Ltd. | |

### (Production method)

A: Components 1 to 6 were uniformly mixed with a mixer.
B: Components 7 to 10 were uniformly mixed with a mixer.
C: While stirring, A was gradually added to B and mixed, so that a body lotion was obtained.

The resulting body lotion was easily spread, and was smooth without stickiness or greasiness and creaky feel, with refreshing feel, and excellent in stability.

### [Formulation example 14: Sun-block cream]

A sun-block cream was prepared using the powder obtained in the example.

| (Components) | Blending ratio (g/kg) |
|---|---|
| 1. Crosslinked polyether modified silicone (Note 1) | 30 |
| 2. Crosslinked dimethylpolysiloxane (Note 2) | 20 |
| 3. Alkyl modified branched polyether modified silicone (Note 3) | 10 |
| 4. Neopentyl glycol dioctanoate | 50 |
| 5. Decamethyl cyclopentasiloxane | 175 |
| 6. Ethylhexyl methoxy cinnamate | 60 |
| 7. Dissolved acrylic silicone resin (Note 4) | 100 |
| 8. Caprylylsilane treated zinc oxide fine particles (Note 5) | 200 |
| 9. Powder in Example 1 | 30 |
| 10. 1,3-Butylene glycol | 20 |
| 11. Sodium citrate | 2 |
| 12. Sodium chloride | 5 |
| 13. Fragrance | appropriate amount |
| 14. Purified water | 297 |

| | |
|---|---|
| (Note 1) KSG-240, manufactured by Shin-Etsu Chemical Co., Ltd. (Note 2) KSG-15, manufactured by Shin-Etsu Chemical Co., Ltd. (Note 3) KF-6038, manufactured by Shin-Etsu Chemical Co., Ltd. (Note 4) KP-575, manufactured by Shin-Etsu Chemical Co., Ltd. (Note 5) Surface treated with AES-3083, manufactured by Shin-Etsu Chemical Co., Ltd. | |

### (Production method)

A: Component 7 was added to a part of component 5 and uniformized. Components 8 and 9 were added thereto and dispersed with Easynano RMB-type bead mill manufactured by AIMEX CO.,LTD.
B: Components 1 to 4, the balance of the component 5, and component 6 were uniformly mixed with a mixer.
C: Components 10 to 12, and component 14 were mixed and uniformized.
D: C was added to B to be emulsified, and A and component 13 were added thereto, so that a sun-block cream was obtained.

The resulting sun-block cream was very easily spread without stickiness, and was excellent in adhesion.

### [Formulation example 15: Pressed cheek color]

A pressed cheek color was prepared using the powder obtained in Example.

| (Components) | Blending ratio (g/kg) |
|---|---|
| 1. Acrylic silicone resin treated mica (Note 1) | 120 |
| 2. Silicone treated talc (Note 2) | 721 |
| 3. Red No. 202 | 3 |
| 4. Yellow iron oxide | 25 |
| 5. Black iron oxide | 3 |
| 6. Silicone treated pigment-grade titanium oxide (Note 2) | 5 |
| 7. Powder in Example 5 | 3 |
| 8. Phenyl modified hybrid silicone composite powder (Note 3) | 20 |
| 9. Dimethylpolysiloxane (6 mm²/s (25°C)) | 50 |
| 10. Vaseline | 20 |
| 11. Polyethylene wax | 30 |

| | |
|---|---|
| (Note 1) Surface treated with KP-574, manufactured by Shin-Etsu Chemical Co., Ltd. (Note 2) Surface treated with KF-9909, manufactured by Shin-Etsu Chemical Co., Ltd. (Note 3) KSP-300, manufactured by Shin-Etsu Chemical Co., Ltd. | |

### (Production method)

A: Components 1 to 8 were uniformly dispersed.
B: Components 9 to 11 were heated and mixed.
C: B was added to A, uniformly mixed with a mixer, and press-molded in a metal plate, so that a pressed cheek color was obtained.

The resulting pressed cheek color was easily spread without greasiness or powderiness, and was excellent in adhesion to the skin with a natural finish.

### [Formulation example 16: Loose powder]

A loose powder was prepared using the powder obtained in Example.

| (Components) | Blending ratio (g/kg) |
|---|---|
| 1. Talc | balance |
| 2. Powder in Example 3 | 10 |
| 3. AMIHOPE LL | 30 |
| 4. PMMA (10 µm) (Note 1) | 80 |
| 5. Preservative | appropriate amount |
| 6. Colorant | appropriate amount |
| 7. Squalane | 10 |
| 8. Preservative | appropriate amount |
| 9. Antioxidant | appropriate amount |
| 10. Fragrance | appropriate amount |

| | |
|---|---|
| (Note 1) Matsumoto Microsphere S-100, manufactured by Matsumoto Yushi-Seiyaku Co., Ltd. | |

### (Production method)

A: Components 1 to 7 were mixed and pulverized.
B: A was transferred into a Henschel mixer, and components 8 to 10 were added thereto. The mixture was stirred to be homogenized.
C: B was pulverized with an atomizer and packed to obtain a loose powder.

The resulting loose powder was excellent in spreadability, and after application, excellent in transparency. Due to the appropriate covering power, no white cast occurred, with a finish exhibiting a natural-looking bare skin feel.

### [Formulation example 17: Eyeliner]

An eyeliner was prepared using the powder obtained in Example.

| (Components) | Blending ratio (g/kg) |
|---|---|
| 1. Black iron oxide | 70 |
| 2. Powder in Example 2 | 50 |
| 3. Vinyl acetate resin emulsion | 450 |
| 4. Concentrated glycerol | 60 |
| 5. Lauric acid POE (20) Sorbitan | 18 |
| 6. Carboxymethyl cellulose (10% aqueous solution) | 180 |
| 7. Purified water | balance |
| 8. Preservative | 1 |
| 9. Fragrance | 2 |

### (Production method)

A: A part of component 5 and component 6 were added to component 7, and components 1 to 3, a part of component 4, and the balance of component 5 were added thereto. The mixture was processed with a colloid mill CM-1 manufactured by Mitsui Electric Seiki Co., Ltd.
B: The balance of component 4 and components 8 and 9 were mixed. A was added to the mixture at 70°C and uniformly dispersed. The dispersion was cooled and packed to obtain an eyeliner.

The resulting eyeliner had uniform color tone, and was excellent in spreadability and adhesion.

### [Formulation example 18: Mascara]

A mascara was prepared using the powder obtained in Example.

| (Components) | Blending ratio (g/kg) |
|---|---|
| 1. Water | 260 |
| 2. Polyvinylpyrrolidone | 20 |
| 3. Butylene glycol | 20 |
| 4. Cationized cellulose (1% aqueous solution) | 100 |
| 5. Bentonite | 5 |
| 6. Triethanol amine | 17 |
| 7. Talc | 27 |
| 8. Powder in Example 6 | 10 |
| 9. Yellow iron oxide | 9 |
| 10. Red iron oxide | 9 |
| 11. Black iron oxide | 48 |
| 12. Carnauba wax | 55 |
| 13. Beeswax | 90 |
| 14. Stearic acid | 20 |
| 15. Self-emulsifying glyceryl stearate | 20 |
| 16. Propylene glycol stearate | 20 |
| 17. Hydrogenated polyisobutene | 20 |
| 18. Cyclomethicone | 40 |
| 19. Preservative | appropriate amount |
| 20. Antioxidant | appropriate amount |
| 21. Resin emulsion | 200 |

### (Production method)

A: Components 7 to 11 were stirred and mixed with a Henschel mixer.
B: A was added to components 1 to 6, and uniformly dispersed with a stirrer.
C: Components 12 to 20 were heated and dissolved in another container.
D: C was added to B, and after emulsification, the mixture was cooled to 40°C, component 21 was added, and the mixture was cooled to room temperature to obtain a mascara.

The resulting mascara had a uniform color tone, moderate gloss, and excellent adhesion to eyebrows.

### [Formulation example 19: Cream eyeshadow]

A cream eyeshadow was prepared using the powder obtained in Example.

| (Components) | Blending ratio (g/kg) |
|---|---|
| 1. Dissolved acrylic silicone resin (Note 1) | 100 |
| 2. Stearyl modified acrylic silicone resin (Note 2) | 20 |
| 3. Branched polyether modified silicone (Note 3) | 15 |
| 4. Decamethyl cyclopentasiloxane | 203 |
| 5. Isotridecyl isononanoate | 30 |
| 6. Dimethyl distearyl ammonium hectorite | 12 |
| 7. Acrylic silicone resin treated pigment (Note 4) | 100 |
| 8. Powder in Example 3 | 100 |
| 9. Spherical nylon | 30 |
| 10. Talc | 40 |
| 11. Ethanol | 50 |
| 12. Purified water | 300 |

| | |
|---|---|
| (Note 1) KP-545, manufactured by Shin-Etsu Chemical Co., Ltd. (Note 2) KP-561P, manufactured by Shin-Etsu Chemical Co., Ltd. (Note 3) KF-6028P, manufactured by Shin-Etsu Chemical Co., Ltd. (Note 4) Surface treated with KP-574, manufactured by Shin-Etsu Chemical Co., Ltd. | |

### (Production method)

A: Component 1 to 6 were mixed, and components 7 to 10 were further added thereto. The mixture was uniformly mixed and dispersed with a mixer.
B: Components 11 to 12 were mixed.
C: B was added to A to be emulsified to obtain a cream eyeshadow.

The resulting cream eyeshadow was easily spread without greasiness or powderiness, and was excellent in adhesion to the skin.

### [Formulation example 20: Eyeshadow]

An eyeshadow was prepared using the powder obtained in Example.

| (Components) | Blending ratio (g/kg) |
|---|---|
| 1. Silicone treated Talc (Note 1) | 360 |
| 2. Powder in Example 2 | 90 |
| 3. Boron nitride | 90 |
| 4. Mica titanium | 350 |
| 5. Dimethicone 1000 cs | 50 |
| 6. Red No. 202 | 5 |
| 7. Neopentyl glycol dioctoate | 10 |
| 8. Squalane | 40 |
| 9. Preservative | appropriate amount |
| 10. Antioxidant | appropriate amount |

| | |
|---|---|
| (Note 1) Surface treated with KF-99P, manufactured by Shin-Etsu Chemical Co., Ltd. | |

### (Production method)

A: Components 1 to 5 were mixed and pulverized.
B: A was transferred into a Henschel mixer, and premixed components 6 to 10 were added thereto. The mixture was stirred and mixed to be homogenized.
C: B was pulverized with an atomizer and press-molded in an aluminum plate to obtain an eyeshadow.

The resulting eyeshadow had a uniform color tone, and was excellent in spreadability and adhesion to the skin.

### [Formulation example 21: Nail enamel]

A nail enamel was prepared using the powder obtained in Example.

| (Components) | Blending ratio (g/kg) |
|---|---|
| 1. Nitrocellulose (1/2 second) | 100 |
| 2. Modified alkyd resin | 100 |
| 3. Acetyl tributyl citrate | 50 |
| 4. Butyl acetate | 150 |
| 5. Ethyl acetate | 194 |
| 6. Ethanol | 50 |
| 7. Toluene | 340 |
| 8. Ultramarine | 5 |
| 9. Powder in Example 1 | 1 |
| 10. Organically modified montmorillonite | 10 |

### (Production method)

A: Components 8 to 9 were dissolved in a part of components 2 and 3, and the mixture was thoroughly kneaded.
B: The balance of components 2 and 3, component 1, components 4 to 7, and component 10 were added to A and mixed. A container was filled with the mixture, so that a nail enamel was obtained.

The resulting nail enamel had an apparent color equivalent to the applied color, a uniform color tone, and excellent spreadability.

### [Formulation example 22: Polyhydric-alcohol-in-oil-emulsion blusher in solid form]

A polyhydric-alcohol-in-oil-emulsion blusher in solid form was prepared using the powder obtained in Example.

| (Components) | Blending ratio (g/kg) |
|---|---|
| 1. Crosslinked polyglycerol modified silicone (Note 1) | 30 |
| 2. Crosslinked dimethylpolysiloxane (Note 2) | 50 |
| 3. Decamethyl cyclopentasiloxane | 190 |
| 4. Dimethylpolysiloxane (6 mm²/s (25°C)) | 230 |
| 5. Cetyl isooctanoate | 50 |
| 6. Behenyl-modified acrylic silicone resin (Note 2) | 30 |
| 7. Paraffin wax (melting point: 80°C) | 90 |
| 8. Dimethyl distearyl ammonium hectorite | 2 |
| 9. Powder in Example 3 | 100 |
| 10. Acrylic silicone treated red No. 226 (Note 3) | 10 |
| 11. Acrylic silicone treated black iron oxide (Note 3) | 2 |
| 12. Acrylic silicone treated mica (Note 3) | 50 |
| 13. Preservative | appropriate amount |
| 14. Fragrance | appropriate amount |
| 15. 1,3-Butylene glycol. | 160 |

| | |
|---|---|
| (Note 1) KSG-710, manufactured by Shin-Etsu Chemical Co., Ltd. (Note 2) KSG-15, manufactured by Shin-Etsu Chemical Co., Ltd. (Note 3) Surface treated with KP-562P, manufactured by Shin-Etsu Chemical Co., Ltd. | |

### (Production method)

A: Components 1 to 8 were heated to 80°C, and uniformly mixed with a mixer.
B: Components 9 to 12 were uniformly mixed with a mixer. The mixture was added to A and uniformly dispersed.
C: Components 13 and 15 were mixed and heated to 80°C.
D: C was added to B to be emulsified. Component 14 was added thereto, and the mixture was poured into a metal plate to be cooled, so that a polyhydric-alcohol-in-oil-emulsion blusher in solid form was obtained.

The resulting polyhydric-alcohol-in-oil-emulsion blusher in solid form was easily spread without stickiness or greasiness, and was excellent in adhesion to the skin with natural finish.

### [Formulation example 23: Blusher]

A blusher was prepared using the powder obtained in Example.

| (Components) | Blending ratio (g/kg) |
|---|---|
| 1. Talc | balance |
| 2. Sericite | 609 |
| 3. Titanium oxide fine particles | 30 |
| 4. Powder in Example 2 | 20 |
| 5. Colorant | appropriate amount |
| 6. Ethylhexyl methoxycinnamate | 30 |
| 7. Octyl palmitate | 50 |
| 8. Preservative | appropriate amount |
| 9. Antioxidant | appropriate amount |

### (Production method)

A: Components 6 to 9 were heated and dissolved.
B: Components 1 to 5 were mixed with a Henschel mixer. With the mixture, A was mixed.
C: B was pulverized with an atomizer and molded in a medium-sized plate, so that a blusher was obtained.

The resulting blusher was easily spread without greasiness or powderiness, and was excellent in adhesion to the skin with natural finish.

### [Formulation example 24: Creamy lipstick]

A creamy lipstick was prepared using the powder obtained in Example.

| (Components) | Blending ratio (g/kg) |
|---|---|
| 1. Palmitic acid/ethylhexanoic acid dextrin (Note 1) | 90 |
| 2. Dipolyglyceryl triisostearate | 100 |
| 3. Glyceryl trioctanoate | 80 |
| 4. Alkyl modified crosslinked dimethylpolysiloxane (Note 2) | 80 |
| 5. Alkyl modified branched polyglycerol modified silicone (Note 3) | 20 |
| 6. Decamethyl cyclopentasiloxane | 400 |
| 7. 1,3-butylene glycol | 50 |
| 8. Purified water | 176 |
| 9. Red No. 201 | appropriate amount |
| 10. Red No. 226 | appropriate amount |
| 11. Yellow No. 4 | appropriate amount |
| 12. Powder in Example 2 | appropriate amount |
| 13. Mica | appropriate amount |
| 14. Fragrance | appropriate amount |

| | |
|---|---|
| (Note 1) Rheopearl (R) TT, manufactured by Chiba Flour Milling Co., Ltd. (Note 2) KSG-43, manufactured by Shin-Etsu Chemical Co., Ltd. (Note 3) KF-6105, manufactured by Shin-Etsu Chemical Co., Ltd. | |

### (Production method)

A: Components 9 to 12 were mixed into a part of component 2, and dispersed with a three-roll mill BR-100VIII manufactured by AIMEX CO.,LTD.
B: Component 1, the balance of component 2, and components 3 to 6 were heated and uniformly mixed with a mixer.
C: A was added to B, and uniformly mixed with a mixer.
D: Components 7 and 8 were mixed and heated. The mixture was added to C to be emulsified.
E: Components 13 and 14 were added to D, so that a creamy lipstick was obtained.

The resulting creamy lipstick was easily spread without stickiness or greasiness, and was moist with no-feeling of dryness.

### [Formulation example 25: Facial cleansing foam]

A facial cleansing foam was prepared using the powder obtained in Example.

| (Components) | Blending ratio (g/kg) |
|---|---|
| 1. Lauric acid | 30 |
| 2. Myristic acid | 90 |
| 3. Palmitic acid | 80 |
| 4. Stearic acid | 100 |
| 5. Glycerol | 150 |
| 6. 1,3-Butylene glycol | 70 |
| 7. Glyceryl stearate | 15 |
| 8. Preservative | 2 |
| 9. Chelating agent | 1 |
| 10. Water | balance |
| 11. Potassium hydroxide | 60 |
| 12. Cocamidopropyl betaine | 33 |
| 13. Cocoyl glycine potassium | 30 |
| 14. Glycosyl trehalose | 45 |
| 15. Powder in Example 3 | 15 |

### (Production method)

A: Components 1 to 9 were mixed, heated and dissolved.
B: In a separate container, components 10 and 11 were weighed and added to A to be saponified.
C: Components 12 to 15 were added to B. The mixture was uniformly stirred and mixed, and then cooled to room temperature. An appropriate container was then filled with the mixture, so that a facial cleansing foam was obtained.

The resulting facial cleansing foam had an appearance with beautiful white color, and was excellent in foaming and foam retention without impairment of the cleansing properties.

### [Formulation example 26: Lipstick]

A lipstick was prepared using the powder obtained in Example.

| (Components) | Blending ratio (g/kg) |
|---|---|
| 1. Ceresin | 110 |
| 2. Carnauba wax | 10 |
| 3. Glyceryl 2-ethylhexanoate | 160 |
| 4. Mineral oil | 150 |
| 5. Hydrogenated polyisobutene | 200 |
| 6. Methyl phenyl polysiloxane | 205 |
| 7. Octyldodecyl ricinoleate | 50 |
| 8. Red No. 202 | 10 |
| 9. Powder in Example 1 | 70 |
| 10. Mica titanium | 30 |
| 11. Antioxidant | appropriate amount |
| 12. Preservative | appropriate amount |

### (Production method)

A: Components 1 to 12 were heated, mixed, and uniformly stirred.
B: After defoaming, A was poured into a mold, and rapidly cooled to obtain a lipstick.

The resulting lipstick had an apparent color equivalent to the applied color, and excellent spreadability

### [Formulation example 27: Lip gloss]

A lip gloss was prepared using the powder obtained in the example.

| (Components) | Blending ratio (g/kg) |
|---|---|
| 1. Dextrin palmitate | 100 |
| 2. Diisostearyl malate | 440 |
| 3. Liquid paraffin (500 cs) | 426 |
| 4. Preservative | 1 |
| 5. Antioxidant | 1 |
| 6. Mica titanium | 1 |
| 7. Aluminum powder | 1 |
| 8. Powder in Example 3 | 30 |

### (Production method)

A: Components 1 to 5 were heated to 85°C, and uniformly dissolved.
B: Components 6 to 8 were added to A, and uniformly dispersed.
C: A container was filled with B at high temperature, and cooled to room temperature, so that a lip gloss was obtained.

The resulting lip gloss had an apparent color equivalent to the applied color, and excellent spreadability and adhesion.

## Claims

1. A titanium (IV) oxide powder composed of peanut-like twin shaped particles having a major axis diameter, which is the longest part of a single particle, of 300 nm or more and 700 nm or less, a minor axis diameter, which is the maximum part in the direction perpendicular to the major axis, of 100 nm or more and 400 nm or less, a constriction diameter, which is the length in the direction perpendicular to the major axis at the central position of the major axis, of 80 nm or more and 200 nm or less, and a ratio of constriction diameter/minor axis diameter of 0.9 or less.

2. The titanium (IV) oxide powder according to claim 1, wherein the titanium (IV) oxide is rutile-type titanium oxide.

3. The titanium (IV) oxide powder according to claim 1 or 2, wherein the single particle is formed by aggregation of fine rod-like particles having a long axis diameter of 30 nm or more and 350 nm or less and a short axis diameter of 3 nm or more and 12 nm or less into a peanut-like twin shape.

4. The titanium (IV) oxide powder according to claim 1 or 2, wherein in the case where the powder is stirred at 2000 rpm in a mixed solution of trimethylsiloxysilicic acid and cyclopentane siloxane at a mass ratio of 1:1 as medium to obtain a coating material with a powder concentration of 100 g/kg, and a coating film formed therefrom with a bar coater having a film thickness of 18.4 µm is exposed to C-light at a color temperature of 6774 K by a double beam method, the coating film has a total light transmittance of 45.0% or more and 60.0% or less, and a haze value of 90.0% or more.

5. The titanium (IV) oxide powder according to claims 1 or 2, wherein at least a part of the surface of the particles constituting the powder is coated with a layer of an inorganic substance and/or an organic substance.

6. The titanium (IV) oxide powder according to claim 5, wherein at least a part of the surface of the particles constituting the powder is coated with a layer of an inorganic substance comprising one or more of aluminum, silicon, zinc, titanium, zirconium, iron, cerium, and tin.

7. The titanium (IV) oxide powder according to claim 5, wherein at least a part of the surface of the particles constituting the powder is coated with a layer of an organic substance comprising one or more of a silicone compound, a coupling agent, a fluorine compound, and a fatty acid.

8. A cosmetic containing the titanium (IV) oxide powder according to claim 1 or 2.

9. A method for producing the titanium (IV) oxide powder according to claim 1 comprising step 1 of adding hydrochloric acid to a slurry containing an acid-soluble titanium compound, and holding the mixture with a hydrochloric acid concentration controlled to 60 g/L or more and 130 g/L or less at a temperature controlled to 25°C or more and 60°C or less, for 1 hour or more.

10. The production method according to claim 9, wherein in step 1, the hydrochloric acid concentration is controlled to 80 g/L or more and 130 g/L or less, and the temperature is controlled to 35°C or more and 45°C or less.

11. The production method according to claim 9 or 10, further comprising step 2 of controlling the hydrochloric acid concentration and/or the temperature to higher than those in step 1 within a range of hydrochloric acid concentration of 180 g/L or less and a range of temperature of 100°C or less and holding for 1 hour or more, after step 1.

12. The production method according to claim 11, further comprising step 3 of firing after step 2.
